(19) Europäisches Patentamt
European Patent Office
Office européen des brevets

(11) **EP 2 511 364 A1**

(12) **EUROPEAN PATENT APPLICATION**
published in accordance with Art. 153(4) EPC


(43) Date of publication:
**17.10.2012 Bulletin 2012/42**

(21) Application number: **10835714.6**

(22) Date of filing: **10.12.2010**

(51) Int Cl.:
***C12N 1/00*** *(2006.01)*

(86) International application number:
**PCT/JP2010/007202**

(87) International publication number:
**WO 2011/070791 (16.06.2011 Gazette 2011/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.12.2009 JP 2009280842**



(71) Applicants:
- **JGC Corporation**
  **Chiyoda-Ku**
  **Tokyo 100-0004 (JP)**
- **Miyazaki Prefecture**
  **Miyazaki City, Miyazaki 880-0805 (JP)**

(72) Inventors:
- **OKUMURA, Taisei**
  **Higashiibaraki-gun**
  **Ibaraki 311-1313 (JP)**
- **NUMATA, Mamoru**
  **Higashiibaraki-gun**
  **Ibaraki 311-1313 (JP)**
- **KOJIMA, Shuzo**
  **Higashiibaraki-gun**
  **Ibaraki 311-1313 (JP)**
- **NAGATA, Yasuhisa**
  **Higashiibaraki-gun**
  **Ibaraki 311-1313 (JP)**
- **TAHARA, Naoki**
  **Yokohama-shi**
  **Kanagawa 220-6001 (JP)**
- **KUROKI, Hiroyuki**
  **Miyazaki-shi**
  **Miyazaki 880-0303 (JP)**
- **KUKIZAKI, Masato**
  **Miyazaki-shi**
  **Miyazaki 880-0303 (JP)**
- **TANAKA, Tomohiro**
  **Miyazaki-shi**
  **Miyazaki 880-0303 (JP)**

(74) Representative: **Becker Kurig Straus**
**Patentanwälte**
**Bavariastrasse 7**
**80336 München (DE)**


(54) **METHOD FOR CULTURING CELLS OR MICROORGANISMS**


(57) A method for culturing cells or microorganisms by dissolving oxygen or carbon dioxide in a culture solution containing a nutrient, the method including: a step of culturing cells or microorganisms by supplying gas containing oxygen or carbon dioxide to a porous body to generate, in a culture solution, bubbles that have a 50% diameter of 200 μm or less in a volume-based particle size distribution, thereby dissolving oxygen or carbon dioxide in the culture solution, wherein the culture solution contains at least one of a cell-protecting agent for protecting the cells and a protein hydrolysate.


**FIG.2**

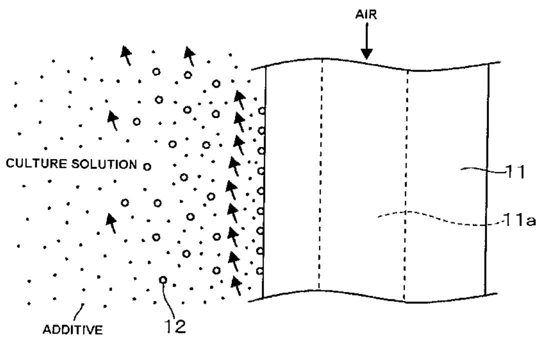


EP 2 511 364 A1

## Description

### FIELD

The present invention relates to a culture method for culturing cells or microorganisms in a culture solution containing a nutrient by dissolving oxygen or carbon dioxide in the culture solution.

### BACKGROUND

As a method for culturing, for example, animal or plant cells or microorganisms, a method is known in which cells or microorganisms are cultured by supplying gas containing oxygen or carbon dioxide, e.g., air, to a culture solution containing a nutrient. This nutrient is, for example, mixed in advance in the culture solution in a predetermined amount, or appropriately supplied into the culture solution during culturing. However, it is necessary that air be continued to be supplied into the culture solution during culturing. In a known method for supplying air into a culture solution, for example, a tubular sparger (e.g., U-shaped sparger) having a large number of gas discharge openings on the leading end side thereof is immersed in the culture solution and bubbles having a diameter of, for example, about several millimeters are supplied into the culture solution.

In this method, in order to further increase the amount of air dissolved in the culture solution, air is made to be easily dissolved in the culture solution by, for example, reducing the size of (breaking up) air bubbles supplied from the sparger by stirring the culture solution with a turbine-type stirring blade so as to increase the contact area between gas and liquid (air and the culture solution). Therefore, in order to further increase the rate (amount) of oxygen dissolution, it is necessary to more vigorously stir the culture solution. Consequently, a large amount of energy is necessary for stirring, and the size and the cost of a apparatus (stirring apparatus) for conducting the culture are also increased. In addition, in the case where the temperature of the culture solution is increased by stirring, cooling energy for decreasing the temperature of the culture solution is necessary. Furthermore, in the case of culturing cells, the cells may be physically damaged by stirring with the stirring blade. Furthermore, the cells may be damaged by an impact caused when the bubbles are broken up (popped) by stirring.

Patent Document 1 describes a technology in which fine bubbles are generated in a liquid through a porous body and these bubbles are utilized in hydroponics, aquaculture of fish and shellfish, foods, microcapsules, pharmaceutical preparations, cosmetics, and the like, and a technology for suppressing proliferation of microorganisms utilizing the fine bubbles, etc. However, the culture of cells or microorganisms has not been studied.

[Patent Document 1] Japanese Laid-open Patent Publication No. 2005-169359 (paragraph 0045)

### SUMMARY

The present invention has been made under the above circumstances, and an object of the present invention is to provide a method for culturing cells or microorganisms wherein when cells or microorganisms are cultured by dissolving oxygen or carbon dioxide in a culture solution containing a nutrient, the oxygen or carbon dioxide can be rapidly dissolved in the culture solution while suppressing stirring of the culture solution to mild or without conducting stirring.

A method for culturing cells or microorganisms by dissolving oxygen or carbon dioxide in a culture solution containing a nutrient, the method including: a step of culturing cells or microorganisms by supplying gas containing oxygen or carbon dioxide to a porous body to generate, in a culture solution, bubbles that have a 50% diameter of 200 μm or less in a volume-based particle size distribution, thereby dissolving oxygen or carbon dioxide in the culture solution, wherein the culture solution contains at least one of a cell-protecting agent for protecting the cells and a protein hydrolysate.

The method for culturing cells or microorganisms according to Claim 1, wherein the culture solution has a surface tension of 51.5 dyne/cm or less.

According to the present invention, in culturing cells or microorganisms such as yeast, mold, bacteria, or microalgae by supplying gas containing oxygen or carbon dioxide in a culture solution containing a nutrient, gas containing oxygen or carbon dioxide is supplied to a porous body to generate, in a culture solution, bubbles that have a 50% diameter of 200 μm or less in a volume-based particle size distribution, and the culture solution contains at least one of a cell-protecting agent for protecting cells and a protein hydrolysate. Accordingly, coalescence (aggregation) of bubbles in the culture solution is suppressed by a surface-active action of the protein hydrolysate or the cell-protecting agent, and bubbles having a very small diameter can be obtained. Thus, the contact area between gas and liquid (air and the culture solution) can be further increased. In addition, since the buoyant force of the bubbles can be suppressed so as to be very small, the bubbles can be maintained in the culture solution in a so-called stationary state, as compared with bubbles that have a diameter of, for example, 300 μm or more and that move upwards upon receiving the buoyant force in the related art. Accordingly, since the gas and the culture solution can be brought into contact with each other over a long period of time, oxygen or carbon dioxide can be rapidly dissolved in the culture solution. Furthermore, it is not necessary to conduct stirring vigorously to such an extent that the bubbles are broken up, and thus stirring can be suppressed to be slow or stirring need not be conducted. Accordingly, the size of the entire bio reactor and consumption energy for the culture can be reduced. In particular, in the case where cells are cultured, physical damage to the cells by stirring can be suppressed.

BRIEF DESCRIPTION OF DRAWINGS

These and other objects and features of the present invention will become clearer from the following description of the preferred embodiments given with reference to the attached drawings, wherein:

FIG. 1 is a schematic view illustrating an example of a bio reactor for carrying out a method for culturing cells or microorganisms according to the present invention;
FIG. 2 is a schematic view illustrating a state where bubbles are generated in a culture solution in the bio reactor;
FIG. 3 is a schematic view illustrating a state where bubbles are generated in a culture solution by a method in the related art;
FIG. 4 is a characteristic diagram illustrating results obtained in an Example of the present invention;
FIG. 5 is a characteristic diagram illustrating results obtained in an Example of the present invention;
FIG. 6 is a characteristic diagram illustrating results obtained in an Example of the present invention;
FIG. 7 is a characteristic diagram illustrating results obtained in an Example of the present invention;
FIG. 8 is a characteristic diagram illustrating results obtained in an Example of the present invention;
FIG. 9 is a schematic view illustrating positions of stirring blades and a defoaming blade installed in the bio reactor used in an Example of the present invention;
FIG. 10 is a characteristic diagram illustrating results obtained in an Example of the present invention;
FIG. 11 is a characteristic diagram illustrating results obtained in an Example of the present invention;
FIG. 12 is a characteristic diagram illustrating results obtained in an Example of the present invention;
FIG. 13 is a characteristic diagram illustrating results obtained in an Example of the present invention;
FIG. 14 is a characteristic diagram illustrating results obtained in an Example of the present invention; and
FIG. 15 is a characteristic diagram illustrating results obtained in an Example of the present invention.

DESCRIPTION OF EMBODIMENTS

A culture method for culturing cells or microorganisms such as yeast, mold, bacteria, or microalgae according to an embodiment of the present invention is described with reference to FIGS. 1 and 2. First, an example of a bio reactor used for carrying out this culture method is briefly described. The bio reactor includes a culture tank 21 for storing a culture solution 13 containing a nutrient and a sparger 22 which is an oxygen supply unit that supplies gas containing oxygen, specifically, air in this example, to the culture solution 13 in the culture tank 21 as very small bubbles (microbubbles) 12. The bio reactor is configured so that air is supplied from an oxygen storage unit 23 that stores air or oxygen therein to the sparger 22 through an oxygen supply path 24 and gas (such as carbon dioxide or the air described above) generated from the culture tank 21 is discharged through a discharge path 25 connected to the top surface of the culture tank 21. Reference numerals 31, 32, 33, and 34 in FIG. 1 indicate a needle valve, a pressure gauge, a flow meter, and a ball valve, respectively. These components are arranged so that the supply and the interruption of air to the culture tank 21 and the pressure and the flow rate of air supplied to the culture tank 21 can be controlled by, for example, a controller (not illustrated). Reference numeral 27 in FIG. 1 indicates a motor for gently stirring the culture solution 13 by rotating, around an axis, stirring blades 26 arranged in the culture tank 21 so as to disperse the bubbles 12 in the culture solution 13, the bubbles 12 being supplied from the sparger 22 into the culture solution 13.

The sparger 22 includes, for example, a porous body (porous membrane) 11 configured to have a substantially cylindrical shape so that an inner region 11a thereof is hollow, and is immersed in the culture solution 13. The upper end of the porous body 11 is hermetically connected to the oxygen supply path 24, and the lower end of the porous body 11 is sealed with, for example, a sealing member (not illustrated). As illustrated in the enlarged view on the lower side of FIG. 1, a large number of fine pores 1 each having a pore diameter (PD) d of, for example, 50 $\mu$m or less are uniformly formed over the entire surface of the porous body 11 so that the inner region 11a of the porous body 11 communicates with an outer region of the sparger 22 (i.e., culture solution 13) through the pores 1 at a large number of positions. This porous body 11 is obtained by, for example, mixing volcanic ash shirasu and glass raw materials such as lime (CaO or $CaCO_3$) and boric acid ($H_3BO_3$), melting the resulting mixture at a high temperature, then conducting a heat treatment at about 700°C, and then conducting an acid treatment. Specifically, glass components in the porous body 11 are very uniformly separated into a first glass phase containing silica ($SiO_2$) and alumina ($Al_2O_3$) as main components and a second glass phase containing boron oxide ($B_2O_3$) and calcium oxide (CaO) as main components by the heat treatment. Therefore, after the acid treatment, the porous body 11 in which the very fine pores 1 are uniformly formed is obtained by adjusting the temperature and the time of the heat treatment, the amounts of components added, etc. This porous body 11 is called, for example, shirasu porous glass (SPG) membrane and is produced by SPG Technology Co., Ltd.

The culture solution 13 in the culture tank 21 contains cells 2 or microorganisms, cells 2 in this example, to be cultured and a nutrient serving as nutrition of the cells 2. This nutrient is a basal medium prepared by mixing plural types of amino acids, vitamins, inorganic salts, sugars, etc. in a predetermined ratio. In addition, the culture solution 13 contains, as an additive, at least one of a protein hydrolysate and a cell-protecting agent for protecting the cells 2. Each of these additives has a surface-active action and suppresses coalescence (aggregation) of the fine bubbles 12 supplied from the sparger 22 into the culture solution 13 by the surface-active action. Specific components of these additives are described in detail below.

The protein hydrolysate is a product obtained by hydrolyzing a protein to amino acids and low-molecular-weight peptides. Examples thereof include a hydrolysate of casein, which is a protein derived from cow's milk, polypeptone, peptone, yeast extract, meat extract, and casamino acids. Examples of the method of this hydrolysis include acidolysis, enzymolysis, and self-digestion. Peptone is a generic name of a compound obtained by hydrolyzing an animal protein or a vegetable protein to amino acids and low-molecular-weight peptides. Polypeptone, which is an example of peptone, is a product manufactured by Nihon Pharmaceutical Co., Ltd. and is a powder obtained by decomposing cow's milk casein with an enzyme derived from an animal, followed by purification and drying. Yeast extract is a powder obtained by extracting a water-soluble component of brewer's yeast (Saccharomyces Cerevisiae Meyen), followed by drying. An example of yeast extract is a product (product name: Dried yeast extract D-3) manufactured by Nihon Pharmaceutical Co., Ltd. Casamino acids are products obtained by hydrolyzing a protein to only amino acids using hydrochloric acid, the products being other than peptides. Note that this protein hydrolysate may be used instead of the nutrient described above.

Examples of the cell-protecting agent include Pluronic F68, Daigo's GF21 (growth promoting factor), and serum. Pluronic F68 is a product (CAS No. 9003-11-6) manufactured by BASF Japan Ltd., and is a surfactant that does not have a function as a nutrient component or a cell growth factor but that have a function of protecting the cells 2. Daigo's GF21 is a product manufactured by Nihon Pharmaceutical Co., Ltd. and is a cell growth-promoting factor containing, as a main component, a growth factor in serum (GFS) obtained by purifying bovine serum to remove $\gamma$-globulin. The serum is, for example, fetal calf serum or calf serum, and has not only a function of supplying a nutrient component and a cell growth factor but also a function of a cell-protecting agent that protects the cells 2 from physical stress due to stirring of the culture solution 13 and aeration during the culture of the cells. The amounts of additives for obtaining fine bubbles 12 in the culture solution 13 by the surface-active action are described in Examples below.

Next, a method for culturing cells 2 of the present invention is described. First, cells 2, a nutrient, and at least one of the protein hydrolysate and the cell-protecting agent are charged in the culture tank 21 together with the culture solution 13. Specifically, in the case of serum culture, in addition to the cells 2, for example, the above-described basal medium and either serum or Daigo's GF21 are charged in the culture solution 13. In the case of serum-free culture, in addition to the cells 2, for example, the basal medium, a cell growth factor, and Pluronic F68 are charged. The amount of protein hydrolysate or cell-protecting agent added to the culture solution 13 is such that coalescence (aggregation) of bubbles 12 can be suppressed by the surface-active action of the protein hydrolysate or the cell-protecting agent. Specifically, the amount added is determined so that the surface tension of the culture solution 13 is 51.5 dyne/cm or less. Note that, as described above, the protein hydrolysate may be used instead of the nutrient.

Subsequently, gas containing oxygen, specifically, air in this example, is supplied from the oxygen supply path 24 to the sparger 22 while controlling the temperature of the culture solution 13 in the culture tank 21 to a predetermined temperature using a heater, a jacket, or the like (not illustrated). The stirring blades 26 are slowly rotated by the motor 27 to disperse bubbles 12 supplied from the sparger 22 to the culture solution 13 in the culture solution 13.

As illustrated in FIG. 2, the air supplied from the sparger 22 into the culture solution 13 is pushed out as a large number of very small bubbles (microbubbles) 12 each having a diameter of, for example, 200 $\mu$m or less from the pores 1 into the culture solution 13 through the inner region 11a of the porous body 11, and adheres to an outer surface of the porous body 11, for example. These bubbles 12 may coalesce (aggregate) with each other on the surface of the porous body 11 by, for example, the surface tension of the culture solution 13. However, since the additive having a surface-active action is contained in the culture solution 13 as described above, the action of the surface tension is suppressed to be small, and thus the coalescence is suppressed. Thus, the bubbles 12 are released into the culture solution 13 while maintaining the above fine size thereof. Furthermore, as described above, since the porous body 11 is composed of glass and has high wettability with the culture solution 13, the coalescence of the bubbles 12 on the surface of the porous body 11 is further suppressed. In FIG. 2, for the purpose of simplifying the illustration, the bubbles 12 are drawn only on one side of the porous body 11.

The coalescence of the bubbles 12 is also similarly suppressed in the culture solution 13 by the surface-active action of the additive. Accordingly, for example, as illustrated in FIG. 4 described below, the diameters of the bubbles 12 (bubble sizes) in the culture solution 13 become very small and uniform, and thus the bubbles 12 become microbubbles having a 50% diameter (median size) of 200 μm or less in a volume-based particle size distribution. Consequently, the specific surface area of the bubbles 12 is increased to increase the contact area between air (bubbles 12) and the culture solution 13, as compared with the case where bubbles having a size of about several millimeters or 300 μm or more in the related art are supplied in the culture solution 13. Note that the above volume-based particle size distribution is not a particle size distribution determined by counting the number of bubbles 12 but a particle size distribution determined on the basis of the volume of the bubbles 12.

In this case, since the diameters of the bubbles 12 are very small, for example, 200 μm or less, the bubbles 12 are hardly affected by the buoyant force and are substantially in a so-called stationary state in the culture solution 13. Accordingly, the bubbles 12 move upward very slowly in the culture solution 13, and thus the contact time with the culture solution 13 becomes long, as compared with the case where the diameters of the bubbles are large. In addition, since the diameters of the bubbles 12 are very small as described above, the inner pressure of the bubbles 12 (the force of the inner air to dissolve in the culture solution 13) is higher than that of bubbles each having a diameter of 300 μm or more. Consequently, the bubbles 12 generated in the culture solution 13 are rapidly dissolved in the culture solution 13.

Here, the cells 2 in the culture solution 13 consume oxygen in the culture solution 13 together with the nutrient, and produce, for example, a product and carbon dioxide. Since the amount of cells 2 (the number of individuals) in the culture solution 13 increases with the lapse of time, the amount of oxygen consumed by the cells 2 increases with the continuation of the culture of the cells 2. Accordingly, the amount of oxygen dissolved in the culture solution 13 (dissolved oxygen) may be decreased with the lapse of time. However, since the bubbles 12 are supplied from the sparger 22 into the culture solution 13 as described above, and the bubbles 12 dissolve in the culture solution 13 as described above, oxygen consumed by the cells 2 is compensated for. That is, by supplying the fine bubbles 12 into the culture solution 13, the rate of decrease in the dissolved oxygen concentration in the culture solution 13 becomes slow or the decrease in the dissolved oxygen is suppressed, as compared with the case where bubbles having a large diameter are supplied. Carbon dioxide produced in the culture solution 13 is then discharged from the discharge path 25. At the time when the consumption of the nutrient and oxygen by the cells 2 and the increase in the cells 2 (culture) are conducted for a predetermined time and the nutrient is used up, the cells 2 no longer consume oxygen. Consequently, the dissolved oxygen concentration in the culture solution 13 rapidly increases.

According to the above embodiment, in conducting the culture of the cells 2 in the culture solution 13, very small bubbles 12 having a 50% diameter of 200 μm or less in a volume-based particle size distribution are generated by supplying air to the porous body 11, and at least one of the protein hydrolysate and the cell-protecting agent is incorporated as an additive in the culture solution 13. Accordingly, coalescence (aggregation) of the bubbles 12 in the culture solution 13 is suppressed by a surface-active action of the additive and the bubbles 12 having a very small diameter can be obtained. Thus, the contact area between gas and liquid (the bubbles 12 and the culture solution 13) can be further increased, as compared with bubbles having a diameter of, for example, 300 μm or more. In addition, since the buoyant force of the bubbles 12 can be suppressed so as to be very small, the bubbles 12 can be maintained in the culture solution 13 in a so-called stationary state, as compared with bubbles having the above large diameter. Accordingly, the bubbles 12 and the culture solution 13 can be brought into contact with each other for a long time, and thus oxygen can be rapidly dissolved in the culture solution 13. In addition, the pressure of the inner air of the fine bubbles 12 to dissolve outside the bubbles 12 is higher than that of bubbles having a large diameter. Consequently, oxygen can be more rapidly dissolved in the culture solution 13.

Furthermore, vigorous stirring for breaking up large bubbles is unnecessary in order to obtain the bubbles 12 described above, and it is sufficient that stirring with the stirring blades 26 is gentle stirring for dispersing the bubbles 12 in the culture solution 13. Consequently, since the consumption energy and the size of the motor 27 can be reduced, the costs (operation cost and the cost of the bio reactor) for the culture of the cells 2 can be suppressed. In addition, heat generated by stirring can be suppressed, and thus, for example, the size of equipment for cooling the culture tank 21 and the culture solution 13 can be reduced. Furthermore, since even gentle stirring is sufficient, physical damage to the cells 2 by stirring can be suppressed. In addition, since the bubbles 12 need not be broken up, it is possible to suppress damage to the cells 2 due to an impact caused when the bubbles 12 are popped.

In the case where an additive is added to the culture solution 13, since the culture solution 13 is liquid used for culturing the cells 2, substances other than the protein hydrolysate and the cell-protecting agent, for example, substances harmful to the cells 2 or the culture of the cells 2 cannot be added to the culture solution 13. However, in the present invention, additives beneficial to the culture of the cells 2 can be used. Therefore, oxygen can be rapidly supplied to the culture solution 13 without adversely affecting the culture of the cells 2.

In the case of bubbles in the related art having a diameter of, for example, 300 μm or more, the bubbles receive a large buoyant force in the culture solution 13 and move rapidly upward, and thus a long contact time with the culture solution 13 cannot be secured. Furthermore, in the case where the above additive is not contained in the culture solution 13, even when fine bubbles 12 are generated by using the sparger 22, as illustrated in FIG. 3, the bubbles 12 are immediately coalesced, for example, on the surface of the porous body 11 by the surface tension of the culture solution 13, resulting in the generation of large bubbles. In this case, in order to rapidly dissolve oxygen in the culture solution 13, vigorous stirring for breaking up the large bubbles is necessary. As a result, the consumption energy and the size of the motor 27 may be increased, and the cells 2 may be damaged. In FIG. 3, similarly, the bubbles are drawn only on one side of the porous body 11.

In the above example, the bubbles 12 are dispersed in the culture solution 13 by stirring with the stirring blades 26. Alternatively, for example, in the case where the bubbles 12 are dissolved immediately after being released from the porous body 11, stirring may not be conducted.

In the above example, the cells 2 are cultured by supplying gas containing oxygen, e.g., air. Alternatively, the present invention may be applied when a plant such as plant cells or microalgae is cultured by supplying gas containing carbon dioxide. In this case, since fine bubbles 12 of the gas containing carbon dioxide are generated in the culture solution 13 through the sparger 22, carbon dioxide can be rapidly dissolved in the culture solution 13 as in the example described above. In such a case, a protein hydrolysate and a cell-protecting agent are used as additives added in order to reduce the diameter of the bubbles 12 (in order to reduce the surface tension of the culture solution 13). The amounts of additives added are appropriately determined on the basis of, for example, experiments.

[EXAMPLES]

Next, experiments conducted regarding fine bubbles 12 are described.

(EXAMPLE 1)

First, in the case where a cell-protecting agent (Daigo's GF21) was added to a culture solution 13 for culturing animal cells, a particle size distribution of bubbles 12 generated from the above-described sparger 22 (porous body 11 having a pore diameter d of 1 μm) was measured. The particle diameter was measured using a laser diffraction/scattering particle size distribution analyzer by continuously supplying the culture solution 13, in which the bubbles 12 were generated by the sparger 22, to a flow cell in the particle size distribution analyzer, irradiating the culture solution 13 with a laser beam, and by evaluating diffraction or scattering of the laser beam.

According to the results, in the case where the amount of cell-protecting agent added was 1% by volume, as illustrated in FIG. 4, a 50% diameter in a volume-based particle size distribution was 200 μm or less (124 μm). Accordingly, it is believed that the influence of the buoyant force is very small in the bubbles 12 having this size, as described above. On the other hand, in the case where the amount of cell-protecting agent added was 0.5%, as illustrated in FIG. 5, the 50% diameter was 238 μm. To examine the relationship between the amount of additive added and the diameter of the bubbles 12 obtained, the diameter of the bubbles 12 was measured for various amounts of Daigo's GF21 added. The results illustrated in FIG. 6 were obtained. Accordingly, it was found that, in order to generate bubbles 12 having a diameter of 200 μm or less, the bubbles being believed to be less affected by the buoyant force, it is necessary to add 1% by volume or more of Daigo's GF21.

(EXAMPLE 2)

To examine the correlation between the amount of additive added and the diameter of bubbles 12 generated, experiments were conducted as in Example 1 for various types and amounts of additive added.

As described above, the diameter of the bubbles 12 generated varies depending on the surface tension of the culture solution 13. Accordingly, first, the surface tension of the culture solution 13 for generating fine bubbles 12 having a diameter of 200 μm or less was examined. Specifically, bubbles 12 were generated using the sparger 22 described above in culture solutions 13 containing various amounts of Daigo's GF21 as an additive. The surface tension of each of the culture solutions 13 and the diameter of the bubbles 12 generated were measured. According to the results, as illustrated in FIG. 7, there was a linear correlation between the surface tension of the culture solution 13 and the diameter of the bubbles 12 generated, and it was found that the relationship is represented by a formula (1) below:

$$y = 28.98x - 1292 \cdots (1)$$

From this formula (1), it was found that, in order to generate bubbles 12 having a fine diameter of 200 μm or less as described above, it is necessary to control the surface tension of the culture solution 13 to be 51.5 dyne/cm or less.

Regarding the additives listed in Tables 1 to 3 below, the surface tension of culture solutions 13 was evaluated for various concentrations of each of the additives. In the case where such fine bubbles 12 were believed to be generated (the surface tension was 51.5 dyne/cm or less), the result was denoted by "A". In the case where bubbles 12 having a diameter larger than the above were believed to be generated (the surface tension was more than 51.5 dyne/cm), the result was denoted by "B". Tables 1 to 3 below include the results.

(Table 1)

| Concentration [mg/L] / Component | 1 | 5 | 10 | 50 | 100 | 500 | 1,000 | 5,000 | 10,000 |
|---|---|---|---|---|---|---|---|---|---|
| Polypeptone | B | B | B | B | B | B | B | B | A |
| Yeast extract | B | B | B | B | B | B | B | A | A |

(Table 2)

| Concentration [mg/L] / Component | 0 | 0.1 | 0.25 | 0.5 | 1 | 10 | 100 | 1,000 | 10,000 |
|---|---|---|---|---|---|---|---|---|---|
| Pluronic F68 | B | B | B | B | B | A | A | A | A |

(Table 3)

| Concentration [vol%] / Component | 0 | 0.2 | 0.5 | 1 | 1.5 | 2 | 3 | 5 | 10 |
|---|---|---|---|---|---|---|---|---|---|
| Daigo's GF21 | B | B | B | A | A | A | A | A | A |

From these results, it was found that, in order to obtain bubbles 12 having a diameter of 200 μm or less, the bubbles 12 being believed to be less affected by the buoyant force, it is necessary to adjust the amount of additive added in accordance with the type of additive.

(EXAMPLE 3)

Next, in a culture medium (surface tension: 48.6 dyne/cm) for culturing microorganisms, the relationship between the bubble diameter of bubbles 12 and a pore diameter d of the porous body 11 was measured. The results illustrated in FIG. 8 were obtained. A linear expression that approximates the above relationship was calculated as follows on the basis of the results:

$$y = 3.4x + 17.5 \cdots (2)$$

(where x represents the pore diameter of the porous body 11 and y represents the diameter (50% diameter) of the bubbles 12.) The $R^2$ value in this case is 1.0. Thus, it is found that the pore diameter d of the porous body 11 can be calculated by the formula (2) from the diameter of the bubbles 12 in the culture solution 13 with a very high accuracy. Accordingly, the pore diameter d of the porous body 11 corresponding to the diameter (200 μm) of the bubbles 12, which are believed to be hardly affected by the buoyant force, was calculated. According to the result, the pore diameter d was 50 μm. Thus, fine bubbles 12 which are hardly affected by the buoyant force can be obtained by using a porous body 11 having a pore diameter d of 50 μm or less.

(EXAMPLE 4)

Next, in this experiment, bubbles 12 were generated in a culture solution 13 using the sparger 22 of the present invention or an existing sparger (U-shaped sparger), and an oxygen supply performance in the culture solution 13 was examined. Specifically, a 3-L mini-jar fermenter manufactured by KK. Takasugi Seisakusho and having an inner diameter of 130 mm and a height of 260 mm (model: TSC-M3L) was used as a culture tank, and, as illustrated in FIG. 9, stirring blades and a defoaming blade, each of which was a six-flat-blade turbine having an outer diameter of 55 mm, were set in the culture tank. Air was supplied into the culture solution 13 at a flow rate of 150 mL/min using each sparger, and a $K_La$ value (overall volumetric oxygen transfer coefficient), which is an indicator of an oxygen dissolving capacity, was measured. According to the results, as illustrated in FIG. 10, in the case where the porous body 11 (SPG membrane) was used, a high dissolved oxygen concentration was obtained even by gentle stirring. In contrast, in the case of the existing sparger (having a U-shape), a high dissolved oxygen concentration was not obtained even when the culture solution 13 was vigorously stirred, and the dissolved oxygen concentration was 1/10 or less of that in the case where the porous body 11 was used. Therefore, in the case of the existing sparger, it is necessary to break up the bubbles by stirring in order to obtain a high dissolved oxygen concentration. For example, in order to obtain a $K_La$ value of 30 $h^{-1}$, 250 rpm of the number of revolutions of the stirring blades 26 was enough in the porous body 11, whereas vigorous stirring at 550 rpm was necessary in the exiting sparger. Thus, the number of revolutions of the stirring blades 26 can be reduced to a half or less by using the porous body 11.

(EXAMPLE 5)

In this experiment, colon bacillus (Escherichia coli, NBRC3301), which is a microorganism, was actually cultured using the sparger 22 (porous body 11) of the present invention or the existing sparger (having a U-shape), and the cell concentration (OD600) was examined. The amount of air supplied was set to 150 mL/min in each case. According to the results, as illustrated in FIG. 11, it was found that, in the case where the porous body 11 was used, after eight hours from the start of the culture, the cell concentration was higher, by about 50%, than that in the case where the existing sparger was used. It is believed that this result corresponds to the diameter of the bubbles 12 supplied to the culture solution 13. Specifically, the larger the specific surface area of the bubbles 12 and the higher the oxygen supply rate, the higher the cell concentration can be.

(EXAMPLE 6)

Furthermore, colon bacillus was actually similarly cultured using the porous body 11 or the existing sparger, and the dissolved oxygen concentration was measured. According to the results, as illustrated in FIG. 12, in the case where the existing sparger was used, the dissolved oxygen concentration was decreased immediately after the start of the culture. On the other hand, in the case where the porous body 11 was used, the decrease in the dissolved oxygen concentration was suppressed.

(EXAMPLE 7)

In Example 6, the dissolved oxygen concentration in the culture solution 13 was measured. In Example 7, the oxygen concentration in gas released (discharged) from the culture solution 13 during culture of colon bacillus was measured. When oxygen in air supplied dissolves in the culture solution 13 and is consumed by colon bacillus, the amount of oxygen discharged decreases. On the other hand, in the case where oxygen does not dissolve in the culture solution 13, the oxygen is discharged directly from the culture solution 13. Thus, whether oxygen is effectively used or not was examined by measuring the oxygen concentration in the discharge gas.

According to the results, as illustrated in FIG. 13, in the case where the porous body 11 was used, the oxygen concentration in the discharge gas decreased. Accordingly, it was found that oxygen dissolved in the culture solution 13, and the oxygen was consumed by colon bacillus. On the other hand, it was found that, in the case where the existing sparger was used, only a relatively small amount of oxygen dissolved in the culture solution 13 and most of the oxygen was discharged. Each of the results was calculated as an effective utilization ratio of oxygen (the amount of oxygen consumed/the amount of oxygen supplied). In the case where the porous body 11 was used, the effective utilization ratio of oxygen was 86%. In the case where the existing sparger was used, the effective utilization ratio of oxygen was 30%. Accordingly, it was found that, by using the porous body 11, the diameter of the bubbles 12 was decreased and air was easily dissolved in the culture solution 13. However, in the case where the existing sparger was used, since the bubbles had a large diameter, most of the supplied oxygen was discharged from the culture solution 13.

(EXAMPLE 8)

Next, in this experiment, Chinese hamster ovary cells (Life Technologies Japan Ltd., Catalog No. 11619-012), which are animal cells, were actually cultured using the sparger 22 (porous body 11) of the present invention or an existing sparger (sintered metal), and the living cell concentration and the glucose concentration were examined. Specifically, a total 7-L animal cell culture tank (Model: BCP-07NP3) manufactured by ABLE Corporation was filled (charged) with 5 L of CHO-S-SFM II, which is a serum-free culture medium manufactured by Life Technologies Japan Ltd., and the Chinese hamster ovary cells were cultured. The living cell concentration was measured with a Thoma Hemocytometer (Model: A105) manufactured by Sunlead Glass Corp. using a solution prepared by adding a 0.05 wt (weight) % nigrosine solution, which is a reagent for determining life and death of cells, to a culture solution sampled from the culture tank. The glucose concentration was measured by a high-performance liquid chromatograph manufactured by Shimadzu Corporation (Column model No.: Shim-pack SPR-Pb 250L x 7.8). The amount of air supplied to the sparger 22 (sparger) was controlled so that the dissolved oxygen concentration of the culture solution was 6.31 mg/L in each of the case of the sparger 22 of the present invention and the case of the existing sparger. According to the results, regarding the living cell concentration, it was found that, as illustrated in FIG. 14, the maximum living cell concentration in the case where the porous body 11 was used was higher, by about 75%, than that in the case where the existing sparger was used. Regarding the glucose concentration, as illustrated in FIG. 15, the initial values were substantially the same, and the decreasing tendencies of the concentration during culture were also similar to each other. Accordingly, it was found that when the bubbles 12 generated from the porous body 11 were present in the culture solution 13 to supply oxygen, the cells 2 could more efficiently consume glucose and proliferate, as compared with the case where bubbles generated from the existing sparger were present.

## Claims

**1.** A method for culturing cells or microorganisms by dissolving oxygen or carbon dioxide in a culture solution containing a nutrient, the method comprising:

a step of culturing cells or microorganisms by supplying gas containing oxygen or carbon dioxide to a porous body to generate, in a culture solution, bubbles that have a 50% diameter of 200 μm or less in a volume-based particle size distribution, thereby dissolving oxygen or carbon dioxide in the culture solution,
wherein the culture solution contains at least one of a cell-protecting agent for protecting the cells and a protein hydrolysate.

**2.** The method for culturing cells or microorganisms according to Claim 1, wherein the culture solution has a surface tension of 51.5 dyne/cm or less.

**3.** The method for culturing cells or microorganisms according to Claim 1 or 2, wherein the porous body has a pore diameter of 50 μm or less.

# FIG.1

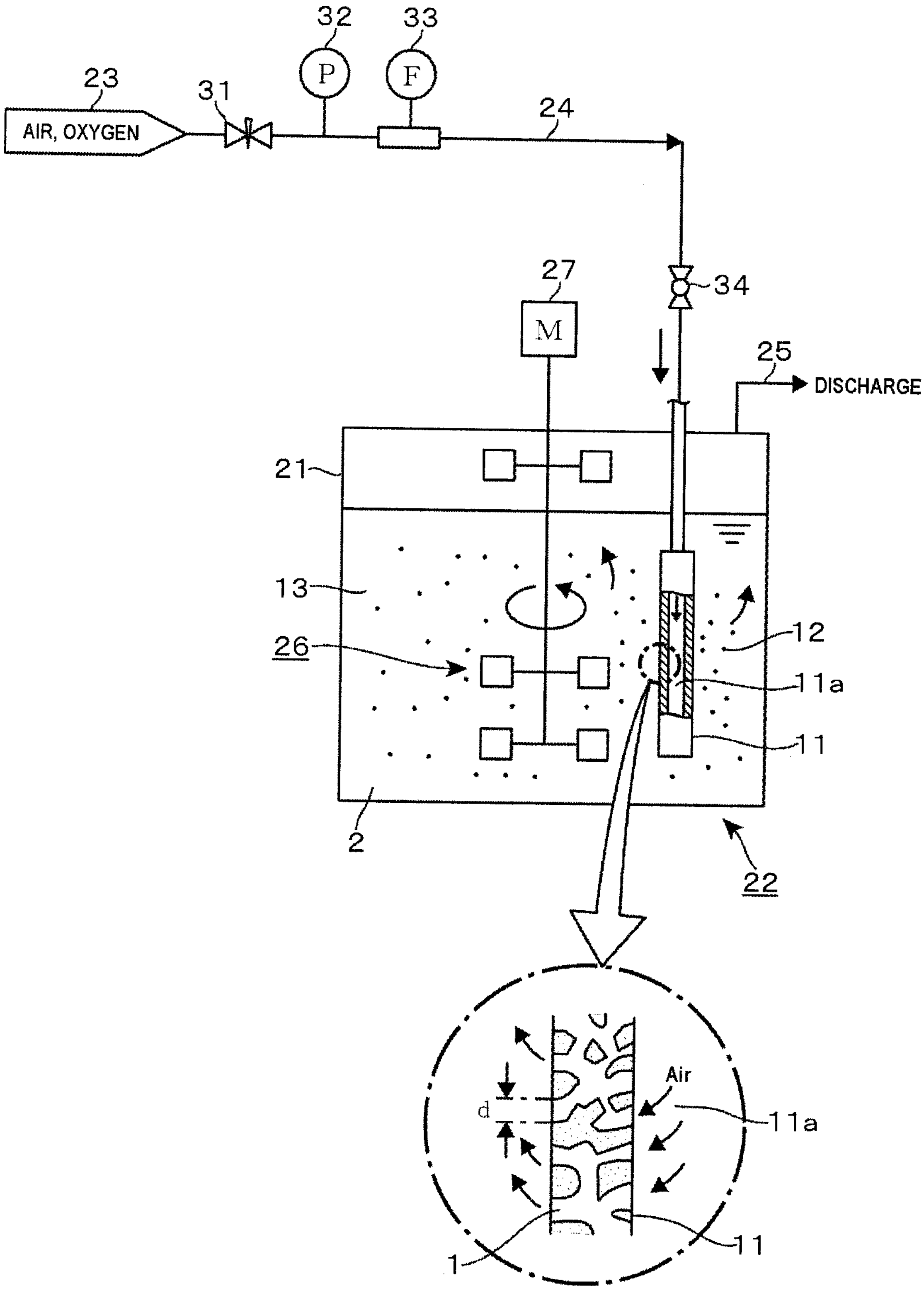

23
AIR, OXYGEN
31
32
P
33
F
24
27
M
34
25
DISCHARGE
21
13
26
12
11a
11
2
22
Air
d
11a
1
11

FIG.2

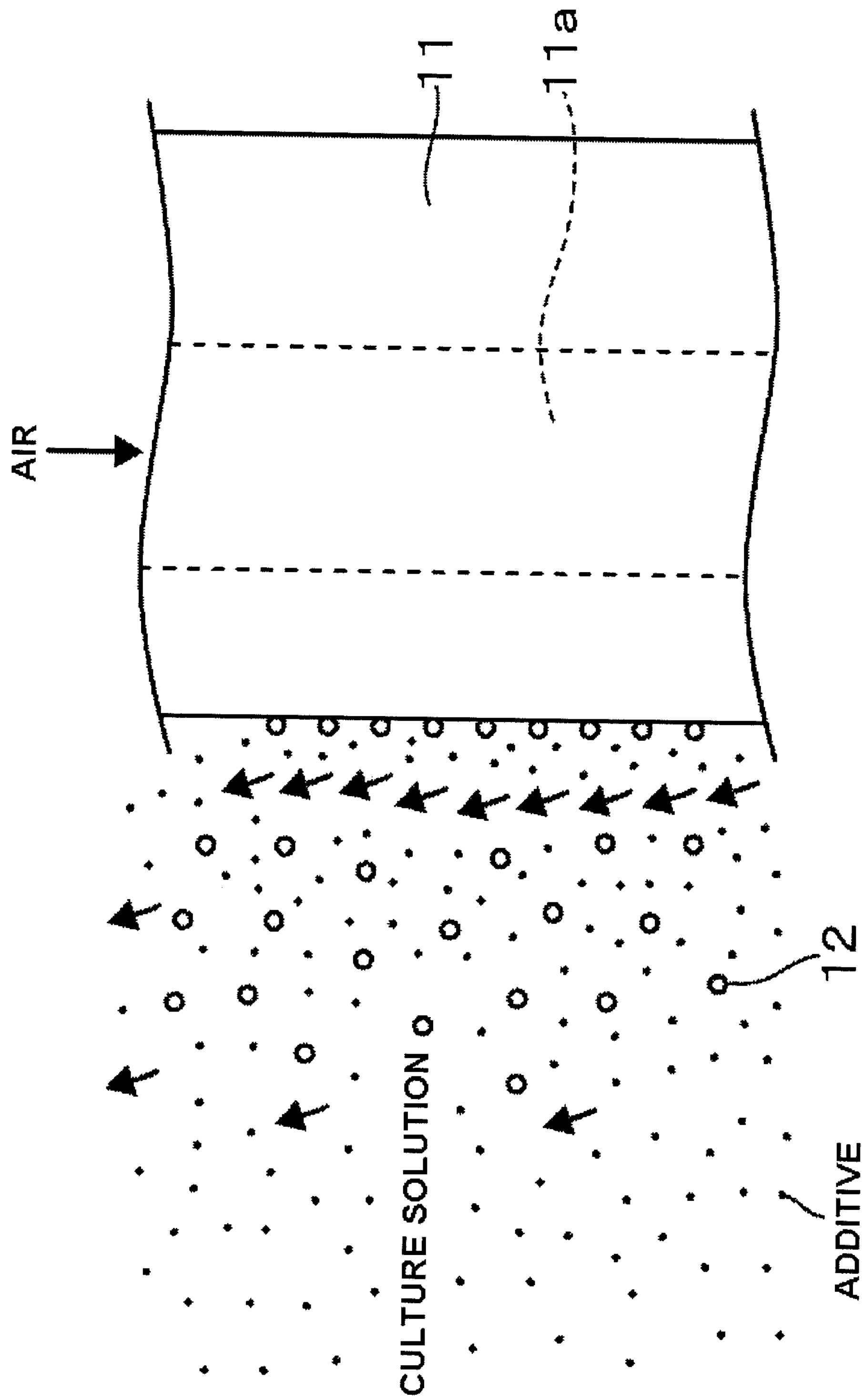

FIG.3

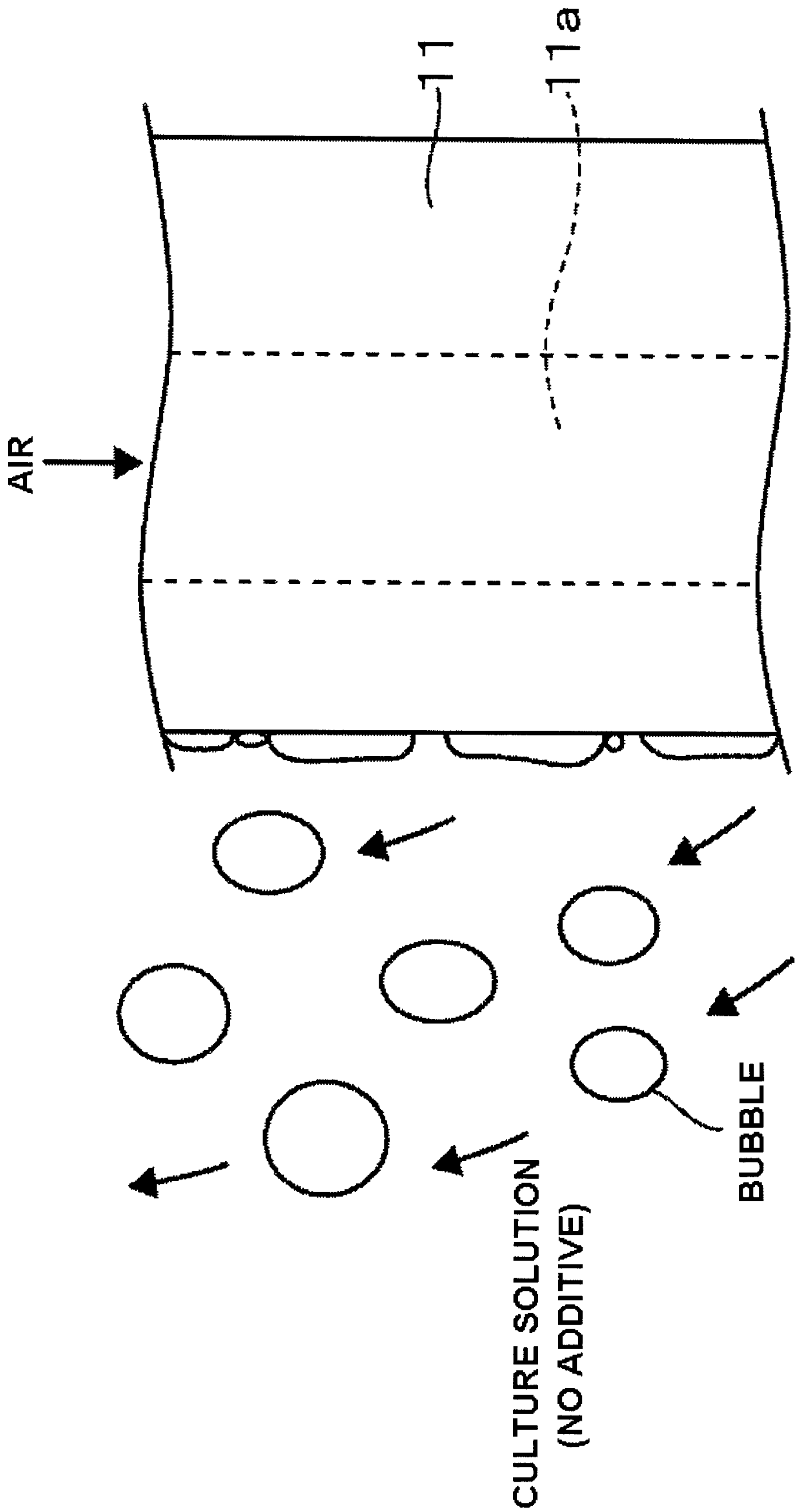

# FIG.4

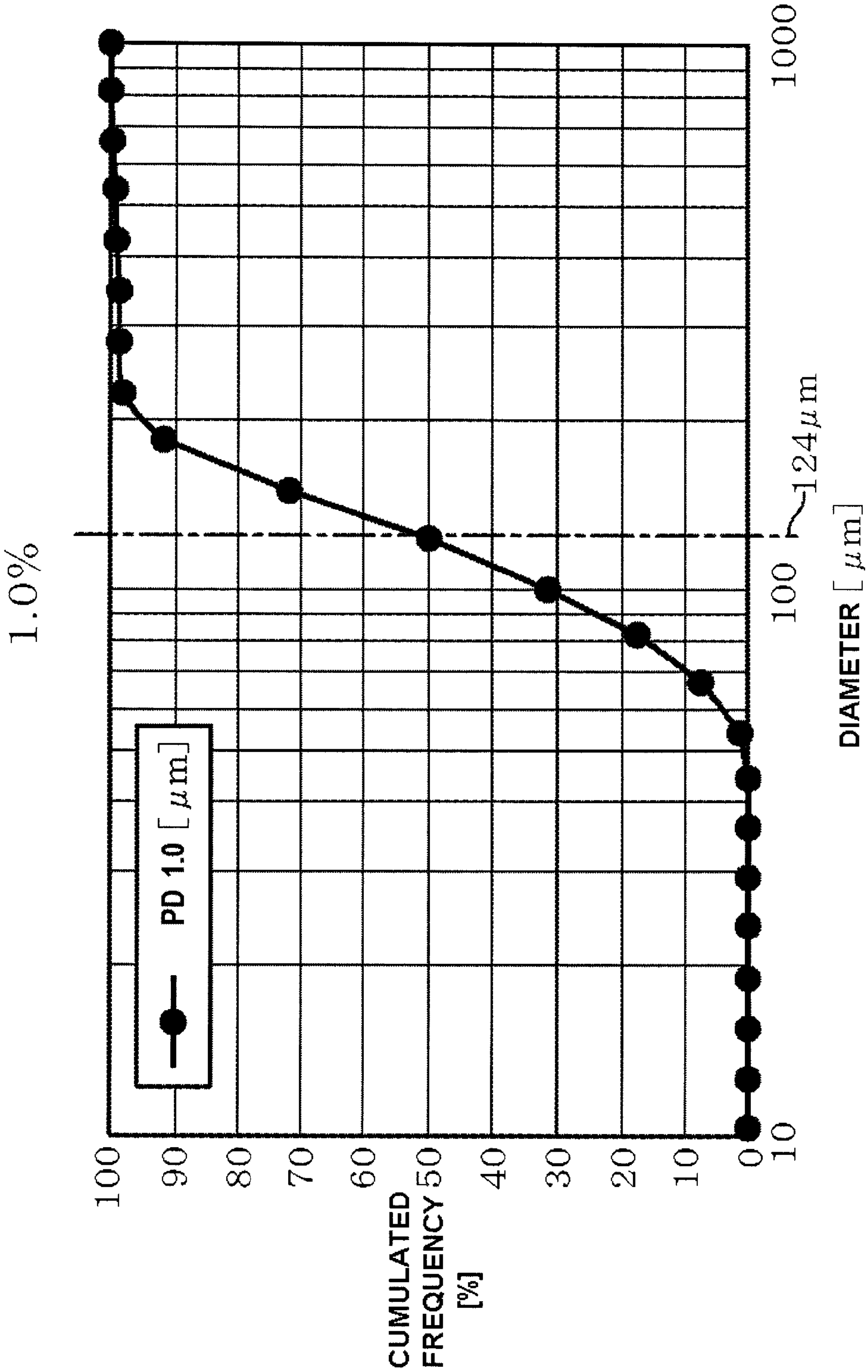
1.0%
PD 1.0 [μm]
124μm
DIAMETER [μm]
CUMULATED FREQUENCY [%]
100
90
80
70
60
50
40
30
20
10
0
10
100
1000

FIG.5

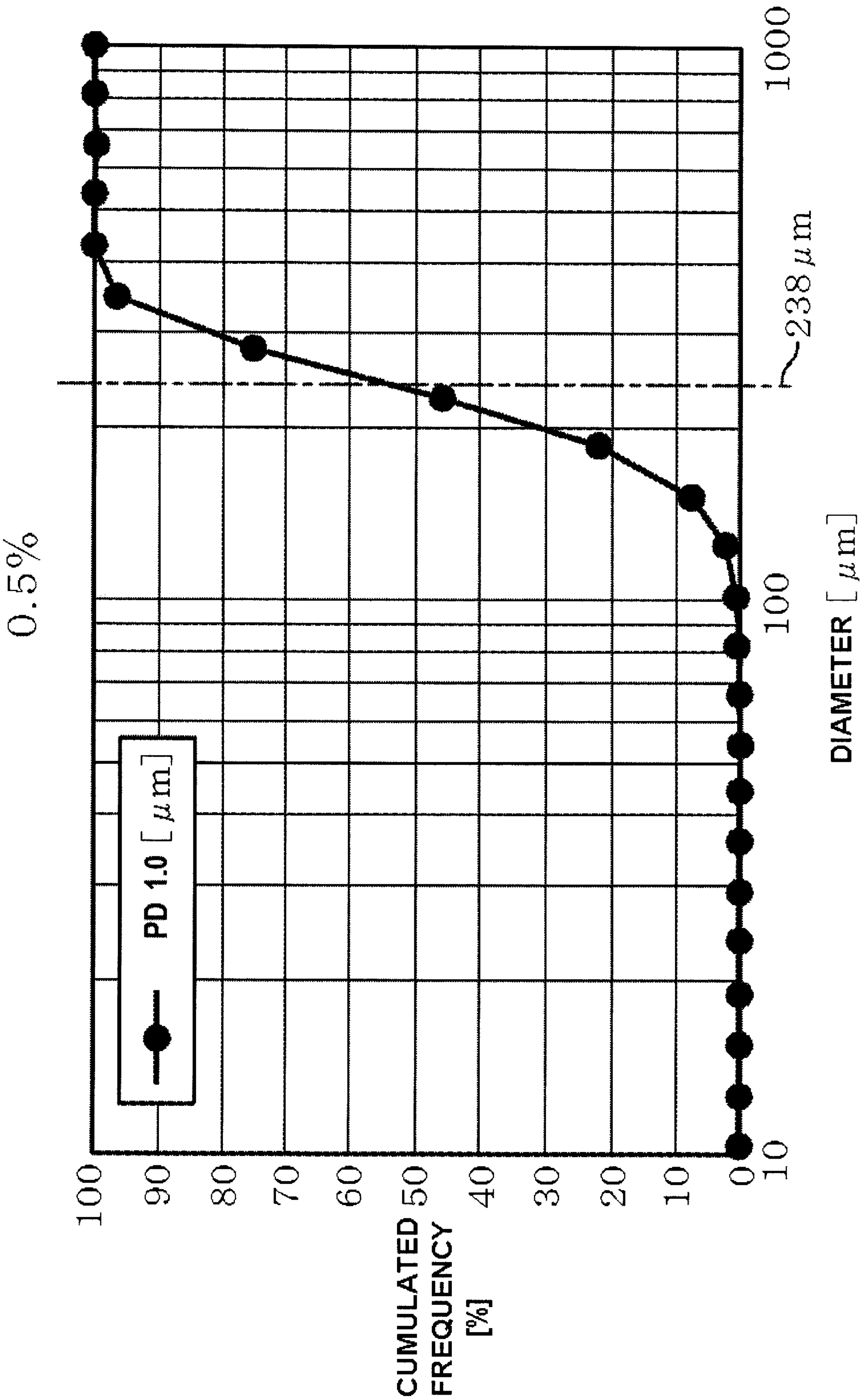
0.5%
PD 1.0 [μm]
238μm
CUMULATED FREQUENCY [%]
DIAMETER [μm]
100
90
80
70
60
50
40
30
20
10
0
10
100
1000

FIG.6

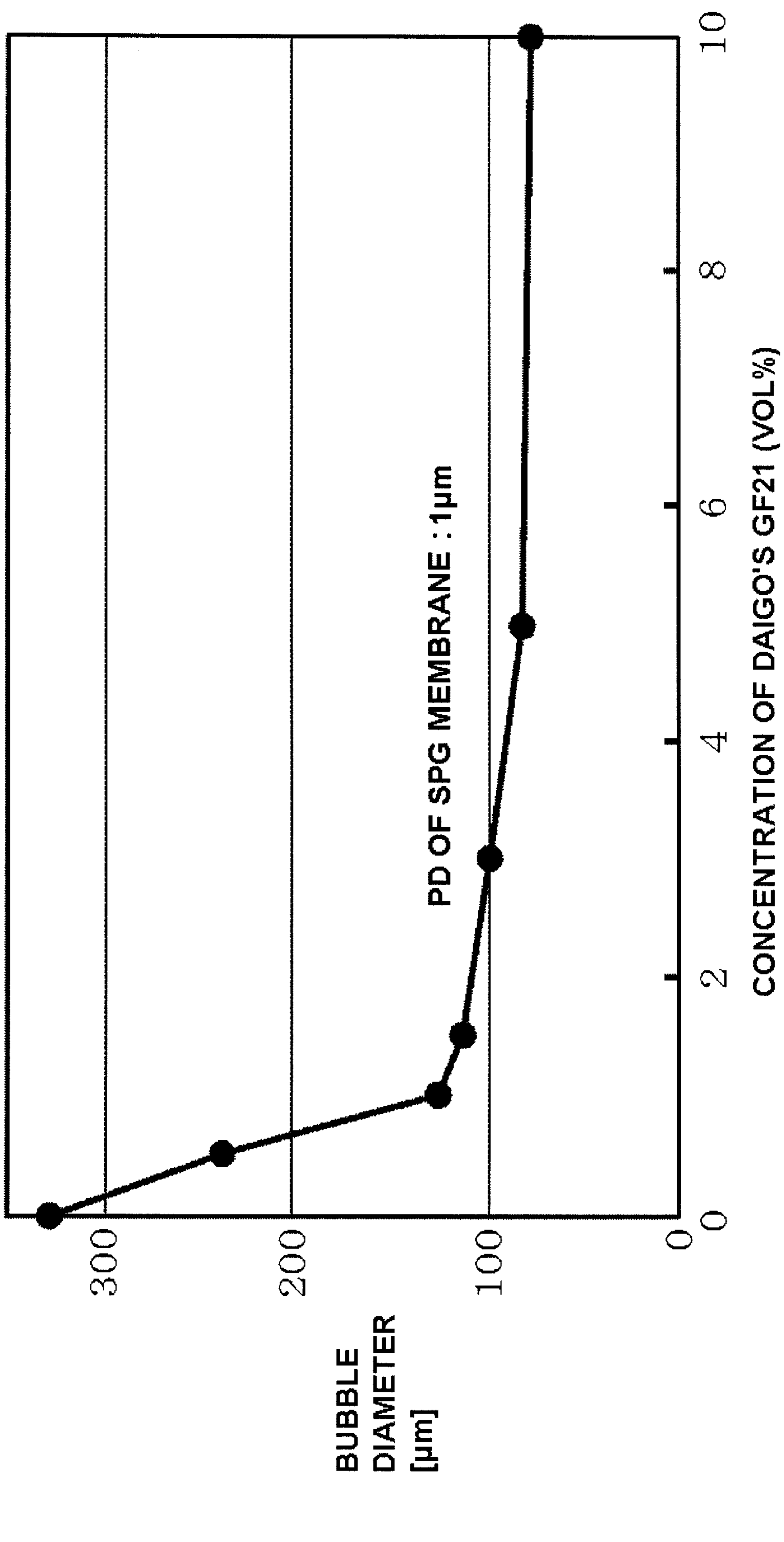
BUBBLE DIAMETER [μm]
300
200
100
0
PD OF SPG MEMBRANE : 1μm
0
2
4
6
8
10
CONCENTRATION OF DAIGO'S GF21 (VOL%)

FIG.7

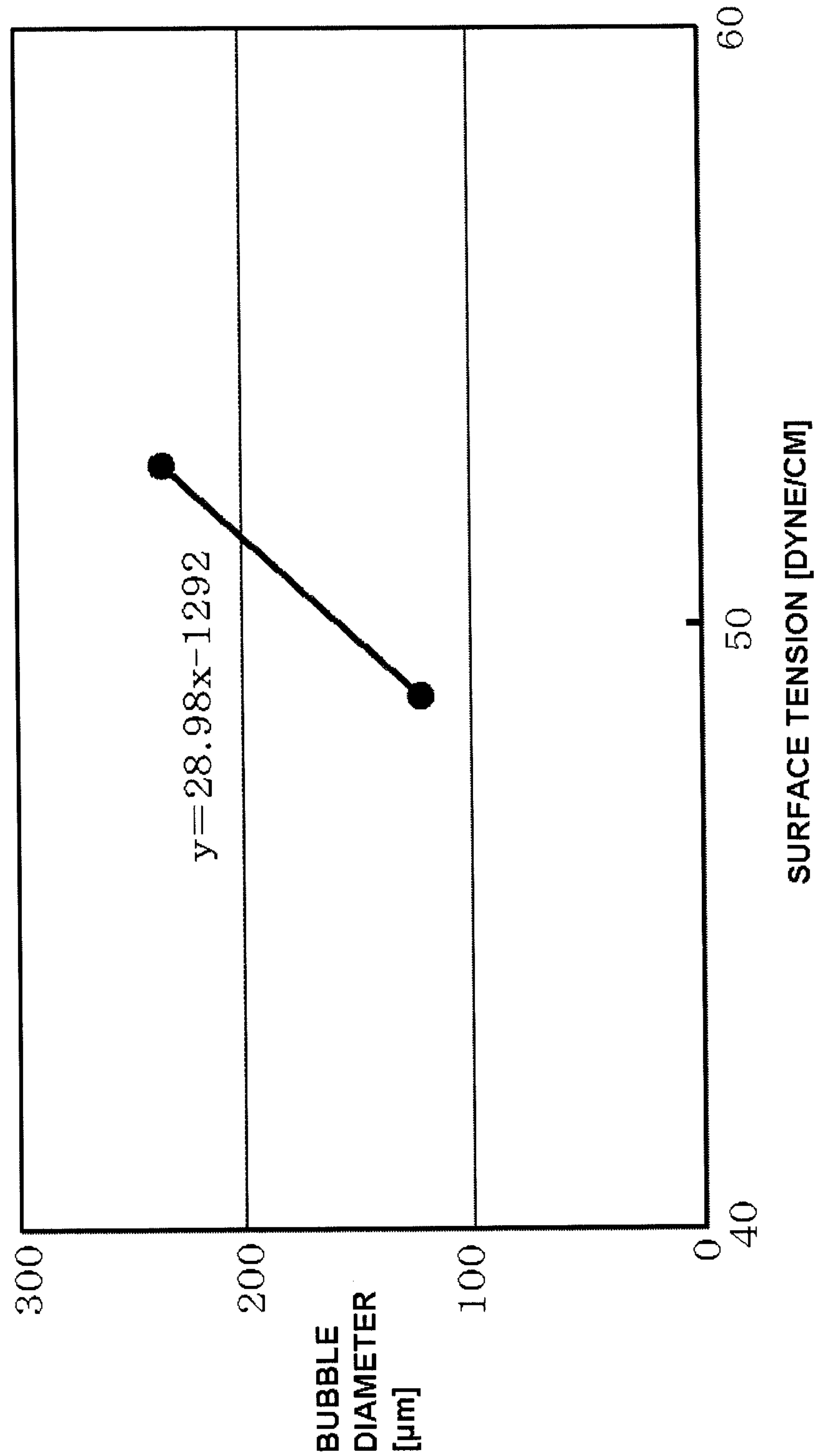
y=28.98x-1292
BUBBLE DIAMETER [µm]
300
200
100
0
40
50
60
SURFACE TENSION [DYNE/CM]

FIG.8

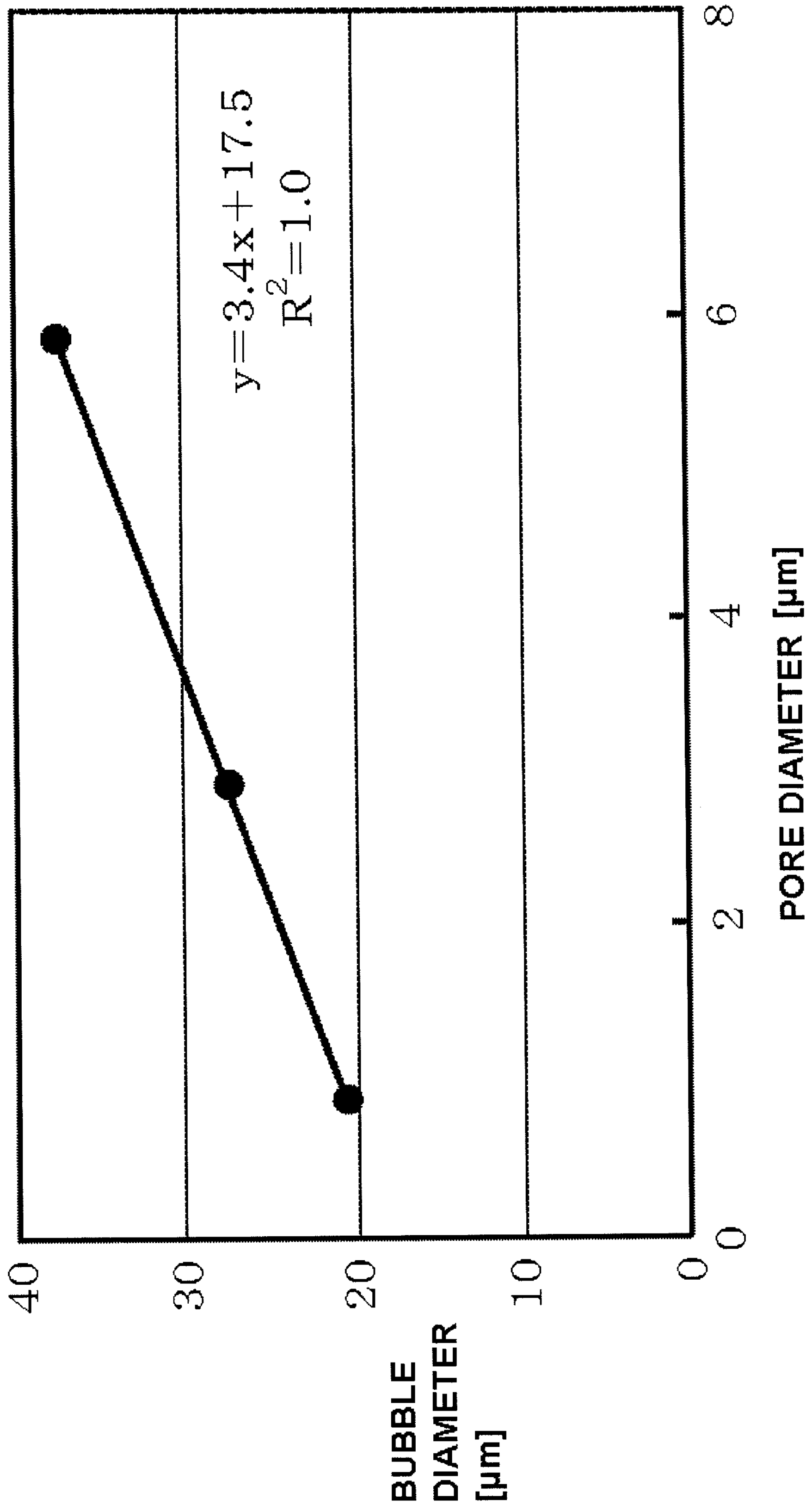
y=3.4x+17.5
$R^2$=1.0
BUBBLE DIAMETER [μm]
PORE DIAMETER [μm]
0
10
20
30
40
0
2
4
6
8

FIG.9

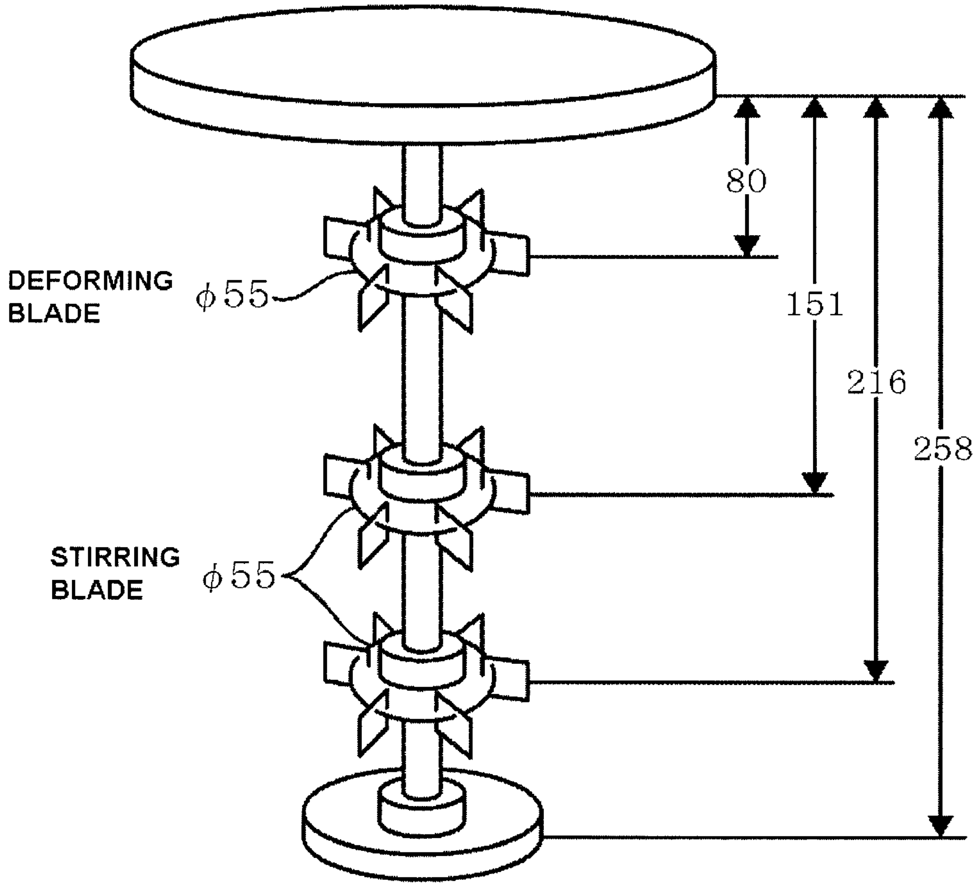

FIG.10

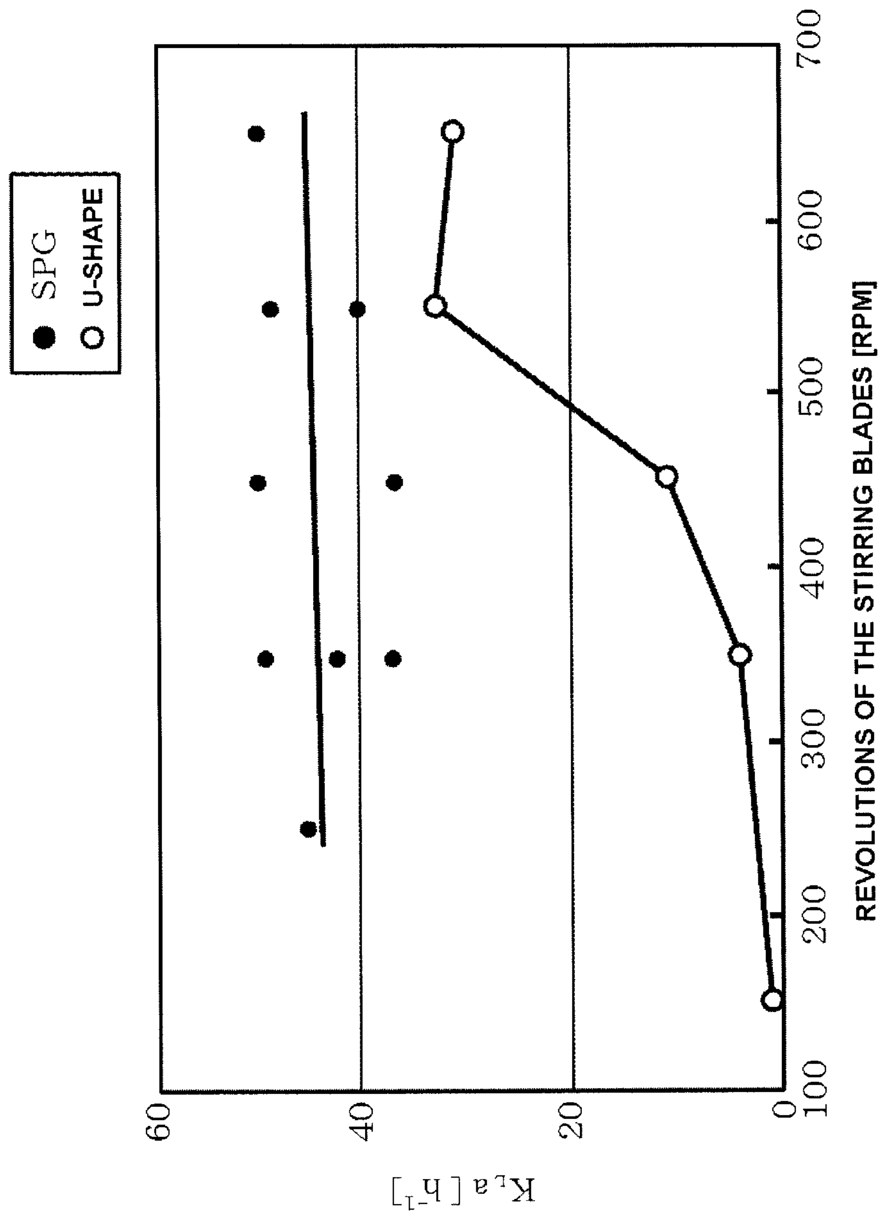
SPG
U-SHAPE
0
20
40
60
K_La [h^-1]
100
200
300
400
500
600
700
REVOLUTIONS OF THE STIRRING BLADES [RPM]

FIG.11

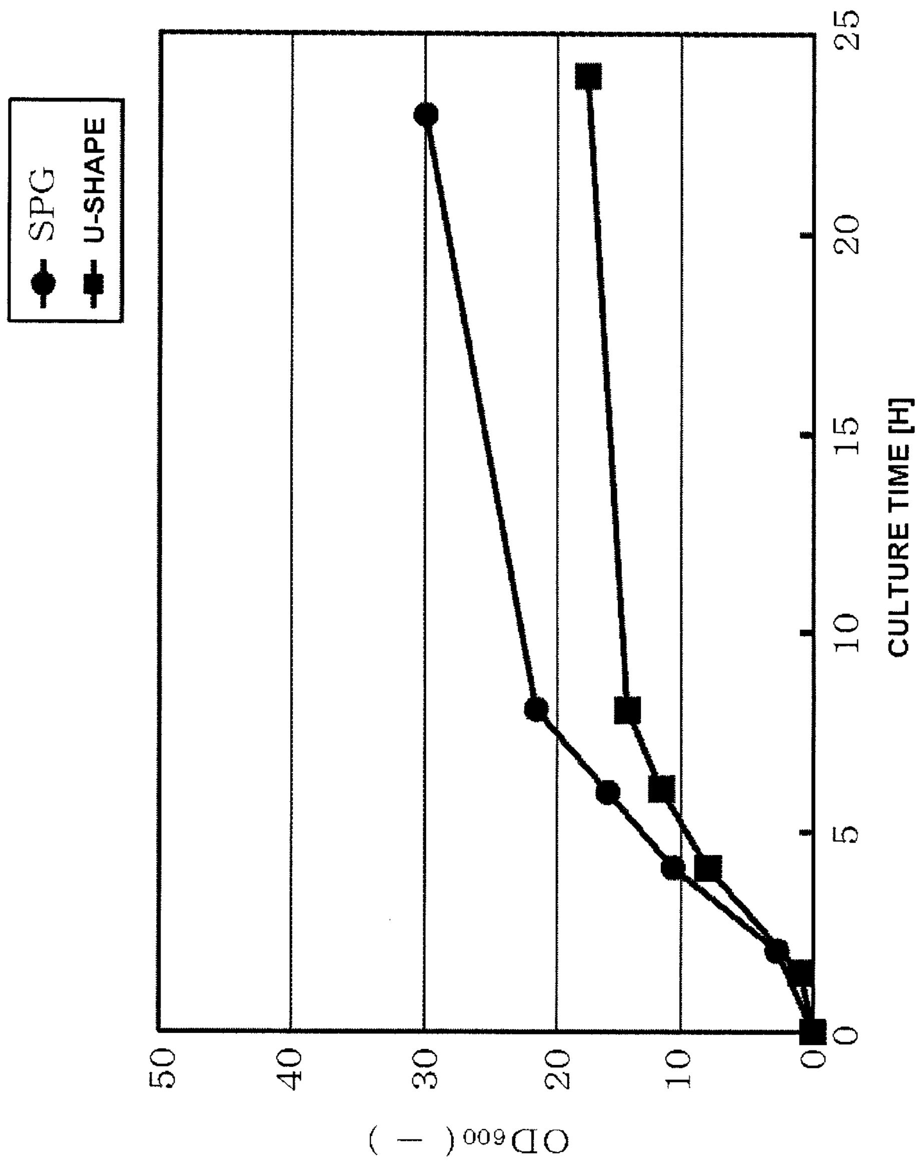

FIG.12

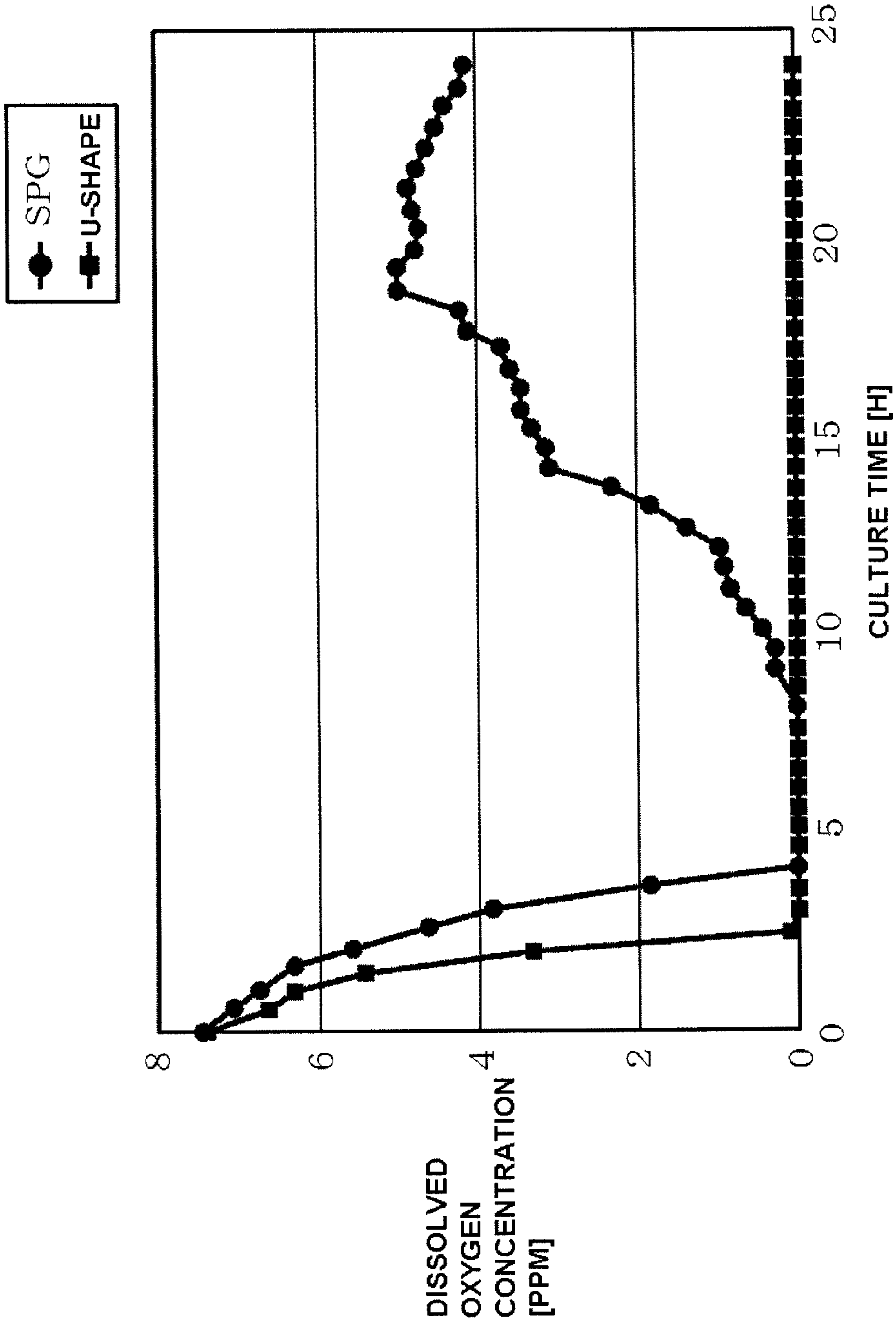
SPG
U-SHAPE
DISSOLVED OXYGEN CONCENTRATION [PPM]
8
6
4
2
0
0
5
10
15
20
25
CULTURE TIME [H]

FIG.13

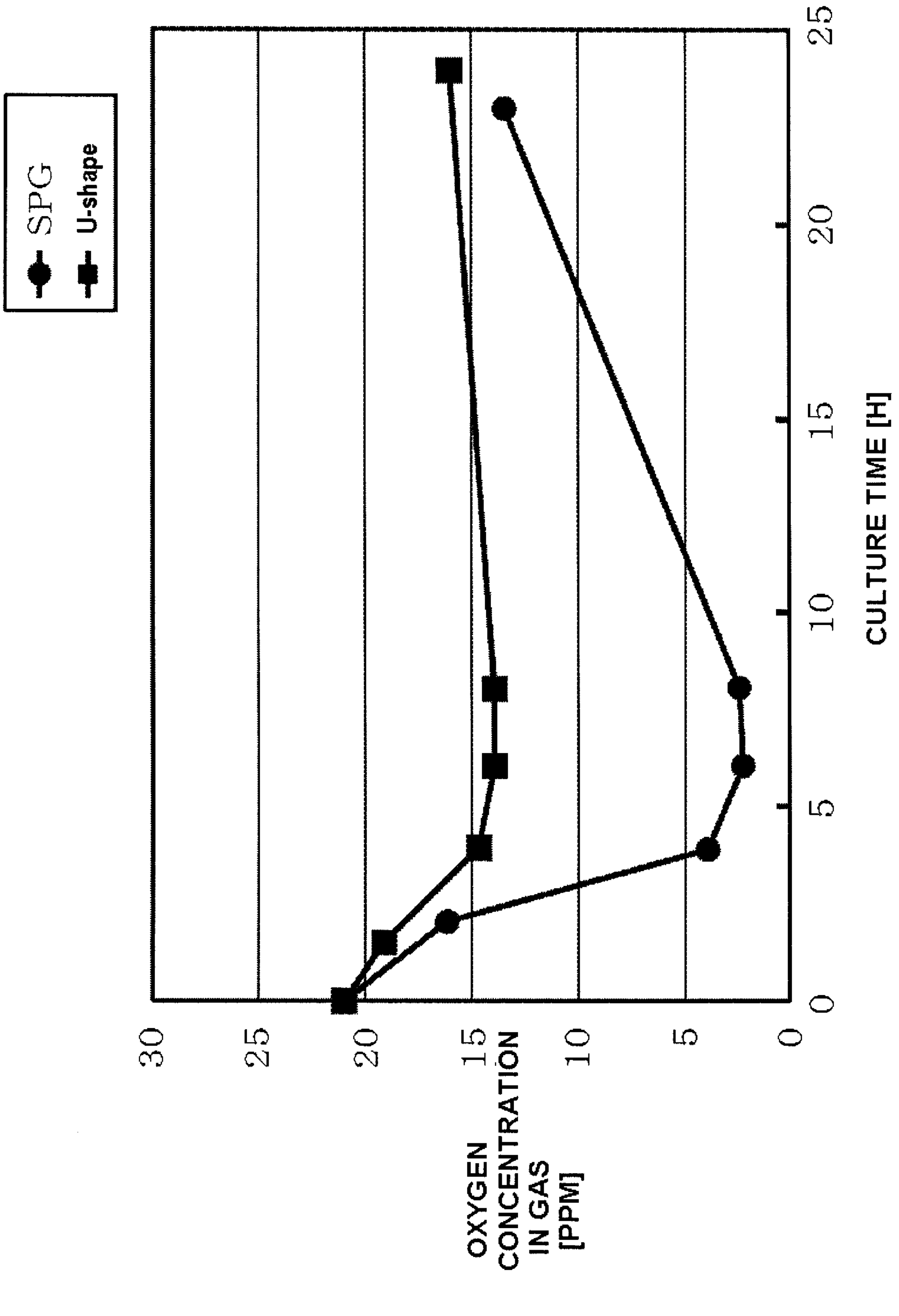
SPG
U-shape
OXYGEN CONCENTRATION IN GAS [PPM]
30
25
20
15
10
5
0
CULTURE TIME [H]
0
5
10
15
20
25

FIG.14

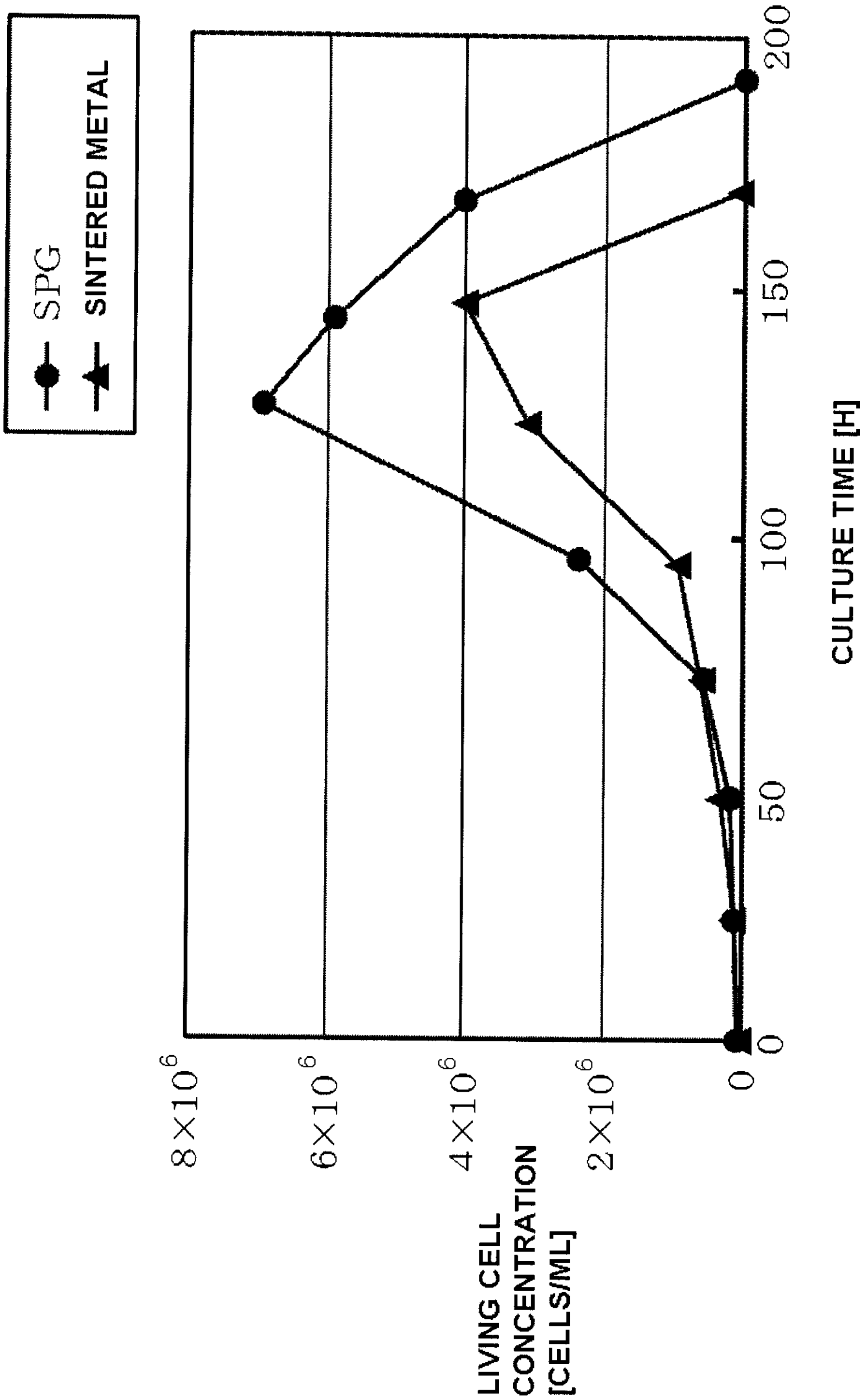
SPG
SINTERED METAL
LIVING CELL CONCENTRATION [CELLS/ML]
8×10^6
6×10^6
4×10^6
2×10^6
0
0
50
100
150
200
CULTURE TIME [H]

FIG.15

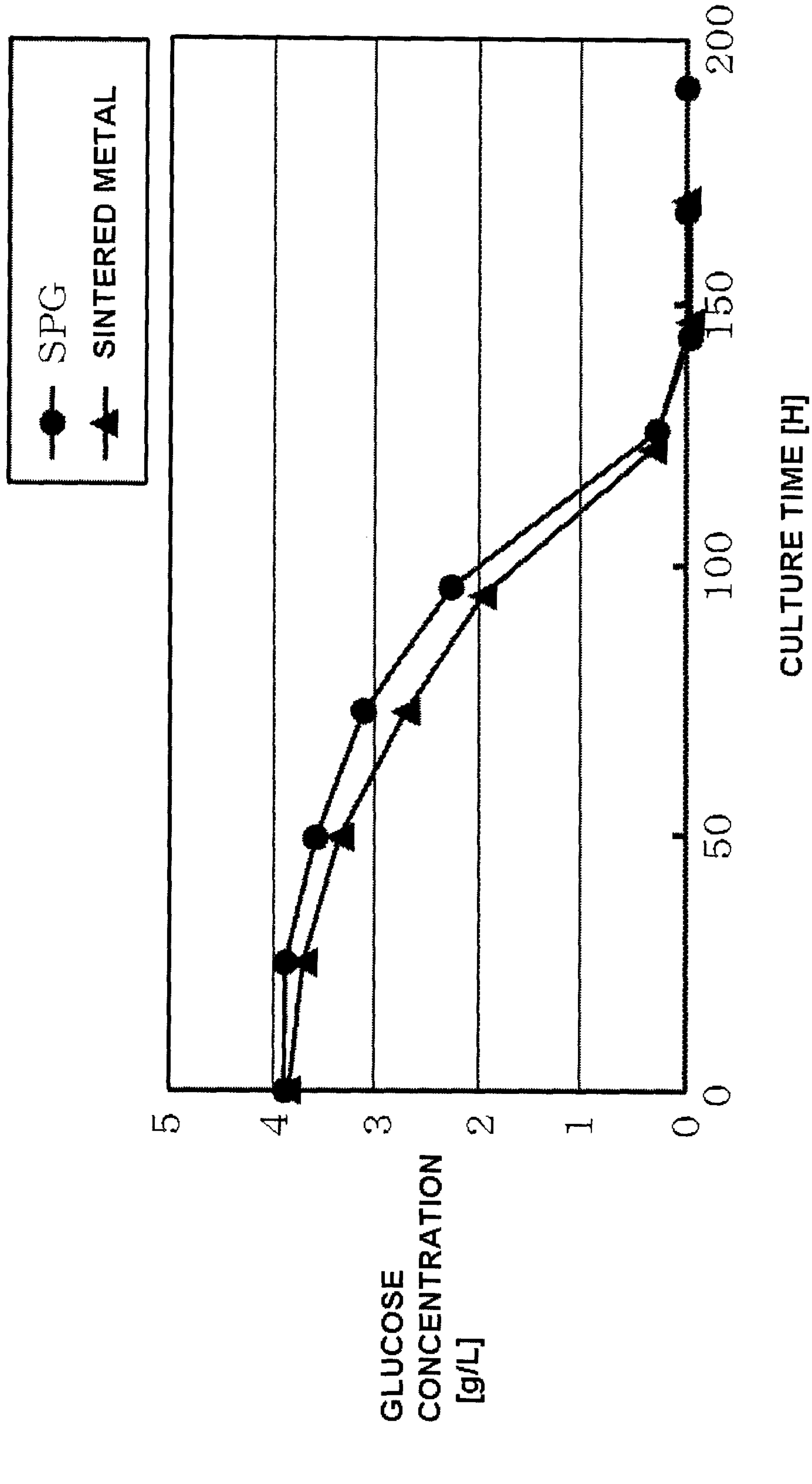
SPG
SINTERED METAL
GLUCOSE CONCENTRATION [g/L]
0
1
2
3
4
5
CULTURE TIME [H]
0
50
100
150
200

## INTERNATIONAL SEARCH REPORT

International application No.
PCT/JP2010/007202

A. CLASSIFICATION OF SUBJECT MATTER
*C12N1/00*(2006.01)i

According to International Patent Classification (IPC) or to both national classification and IPC

B. FIELDS SEARCHED

Minimum documentation searched (classification system followed by classification symbols)
C12N1/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII), G-Search, PubMed

C. DOCUMENTS CONSIDERED TO BE RELEVANT

| Category* | Citation of document, with indication, where appropriate, of the relevant passages | Relevant to claim No. |
|---|---|---|
| X | S.Zhang, et al., Oxygen transfer properties of bubbles in animal cell culture media., Biotechnology and Bioengineering(1992), Vol.40, No.2, p.252-259 | 1-3 |
| Y | JP 2009-018296 A (SPG Trading Kabushiki Kaisha), 29 January 2009 (29.01.2009), paragraph [0005] (Family: none) | 1-3 |
| A | Dirk Nehring, et al., Experimental study of a ceramic microsparging aeration system in a pliot-scale animal cellculture., Biotechnol. Prog.(2004), Vol.20, No.6, p.1710-1717 | 1-3 |

☐ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 February, 2011 (16.02.11) | 01 March, 2011 (01.03.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005169359 A